# FASCICULE DE BREVET EUROPEEN

(11) **EP 0 869 982 B1**
(45) Date de publication et mention de la délivrance du brevet: **19.03.2003**
(21) Numéro de dépôt: 96944090.8
(22) Date de dépôt: 27.12.1996
(51) Int. Cl.: C08G 18/80

(54) **PROCEDE DE MASQUAGE D'ISOCYANATE, UTILISATION D'OXYMES POUR LE MASQUAGE D'ISOCYANATE, ISOCYANATES ET UTILISATION DE CES DERNIERS DANS LA FABRICATION DE REVETEMENT**
VERFAHREN ZUR MASKIERUNG VON ISOCYANAT, VERWENDUNG VON OXIMEN ZUR MASKIERUNG VON ISOCYANAT, MASKIERTE ISOCYANATE UND IHRE VERWENDUNG ZUR HERSTELLUNG VON BESCHICHTUNGEN
ISOCYANATE MASKING PROCEDURE, USE OF OXIMES IN ISOCYANATE MASKING, MASKED ISOCYANATES AND THEIR USE IN MANUFACTURE OF COATING MATERIALS

(30) Priorité: 29.12.1995 FR 9515709
(43) Date de publication de la demande: 14.10.1998
(73) Titulaire: RHODIA CHIMIE, 92408 Courbevoie Cédex (FR)
(72) Inventeur: BERNARD, Jean-Marie, F-69440 Mornant (FR); PERROUD, Eugénie, F-69006 Lyon (FR)
(74) Mandataire: Ricalens, François
(86) Numéro de dépôt international: FR9602090
(87) Numéro de publication internationale: WO97024386

(56) Documents cités:
- EP-A- 0 246 071
- WO-A-94/15984
- CHEMICAL ABSTRACTS, vol. 82, no. 12, 16 Juin 1975 Columbus, Ohio, US; abstract no. 157929a, WAKIMOTO ET AL: "PAINT COMPOSITIONS" page 96; colonne 1; XP002013295 & JP 49 028 255 A (TAKEDA CHEMICAL) 25 Juillet 1974

## Description

La présente invention a pour objet de nouveaux groupes protecteurs thermolabiles des fonctions isocyanates. Elle concerne plus particulièrement un procédé de masquage d'isocyanate, l'utilisation d'oximes pour le masquage d'isocyanate, des isocyanates masqués et l'utilisation de ces derniers dans la fabrication de revêtement.

La présente invention a en particulier pour objet des composés moléculaires constituant une unité, qu'elle soit de nature mono-, oligo- ou poly-mériques, porteuse de groupements isocyanates et susceptible de réagir avec des coréactifs appropriés, tels que les alcools, phénols, amines, aminophénols ou amino-alcools, avantageusement au moins partiellement bi- ou polyfonctionel, qui peuvent être de nature mono-, oligo- ou polymériques.

Plus précisément, la présente invention concerne des polyisocyanates dont certains au moins des groupements fonctionnels isocyanates sont masqués, ou protégés, par des radicaux protecteurs, radicaux qui seront parfois identifiés dans la suite de la description par le qualificatif de "masquant" ou "bloquant".

La présente invention concerne également certains des procédés d'obtention de ces nouveaux polyisocyanates masqués.

Elle vise en outre l'utilisation des polyisocyanates masqués ci-dessus dans des compositions pour la préparation de polymères, notamment de polycondensats et de réticulats issus de la réaction desdits polyisocyanates protégés et de coréactifs nucléophiles. Cette préparation est celle qui est exploitée dans les applications industrielles, telles que les revêtements en tout genre et notamment ceux surs les textiles, sur les verres, sur les papiers, sur les métaux et sur les matériaux de construction, et les peintures.

L'utilité du masquage des fonctions isocyanates (masquage désigné parfois par blocage), voire sa nécessité, s'explique par une -éactivité trop élevée à température ambiante, de l'isocyanates vis à vis de certains coréactifs ou vis à vis d'un solvant réactif, ou d'une phase, en général continue, support dans le cas d'émulsions ou de suspensions, tel que l'eau. Cette réactivité élevée est souvent très gênante notamment pour certaines applications des polyuréthannes, en particulier dans les peintures, car cela impose un conditionnement et parfois une manipulation séparés du co-monomère isocyanate. Il en découle une mise en oeuvre peu commode.

Ainsi, dans toutes les applications des polyuréthannes comme revêtements, il est du plus grand intérêt de disposer d'isocyanates protégés, dans lesquels la fonction isocyanate est rendue non réactive à température ambiante vis-à-vis de ses coréactifs, mais maintenue réactive à une température plus élevée.

Ces unités isocyanates masquées sont avantageuses à plusieurs titres. En premier lieu, elles permettent de proposer, dans un seul et même conditionnement, des compositions (y compris émulsions et suspensions) pour l'obtention de revêtement dont le composant isocyanate soit stable et peu sensible à l'eau. Il s'ensuit qu'il n'est plus nécessaire d'utiliser des solvants anhydres onéreux, spécifiques des isocyanates et qu'il est possible de conserver longtemps, sans dégradation, les isocyanates masqués dans des conditions où ceux qui sont libres se dégraderaient.

Enfin, la mise en oeuvre de polyisocyanates masqués permet de réduire, voire d'éliminer, l'éventuel risque toxique associé à la présence d'isocyanates libres et instables.

L'amélioration de cette technique de masquage des groupements fonctionnels isocyanates sur des unités mono-, oligo- ou polymères réactives, passe par l'optimisation, en général un abaissement, de la température de réaction, c'est-à-dire celle à laquelle la déprotection s'effectue, conduisant ainsi à la polymérisation et/ou la réticulation visées.

Plus spécifiquement la température de démasquage doit être suffisamment haute pour qu'il n'y ait pas de risque de réaction pendant la période de stockage et cette température de réaction doit être suffisamment basse pour qu'il soit aisé de réaliser la polycondensation lorsque cela est désiré.

En général la température de "libération" des isocyanates, notamment aliphatique (c'est-à-dire que le carbone porteur de l'azote est d'hybridation sp3) est trop élevée. Ce qui implique que l'on recherche un abaissement de cette température de libération.

Un tel abaissement se traduit par des gains économiques non négligeables en énergie et en durée de procédé.

A titre incident il convient de signaler que les groupes masquants utilisés dans le cas des isocyanates aromatiques ne sont en générai pas directement transposables pour les isocyanates aliphatiques, la température de "libération" pour un même groupe masquant étant de plusieurs dizaines de degrés centigrades supérieure à celle des isocyanates aromatiques.

De nombreux radicaux bloquants ont déjà été utilisés. Parmi ceux-ci, on peut citer, entre autres, certains des triazoles, des imidazolines, des lactames, des composés hydroxynitrés, des bisulfites de sodium, des dimères d'isocyanate, des phénols, des esters d'acide acétoacétique et des alcools. Un des groupes les plus utilisés est le groupe des dialcoylcétoximes qui présente toutefois l'inconvénient majeur de présenter une température de libération élevée, trop élevée pour beaucoup d'applications.

Parmi ces agents masquants, on ne considere comme de vrais agent masquant que ceux dont le test à l'octanol donne une temperature de déblocacage comprise entre environ 100°C (deux chiffres significatifs) et 180°C (deux chiffres significatifs).

Il convient de noter que la multiplicité des paramètres rend difficile la systématisation de certaines familles.

Ainsi, l'un des objectifs essentiels de la présente invention est de fournir de nouveaux polyisocyanates à groupements fonctionnels bloqués et ayant une température de dissociation relativement basse pour une durée limitée et avec un rendement de dissociation compatible avec les techniques de polymérisation.

Un autre objectif de l'invention est de fournir de nouveaux polyisocyanates à groupements fonctionnels bloqués, qui ne soient que peu ou pas toxiques.

Un autre objectif de l'invention est de fournir de nouveaux polyisocyanates à groupements fonctionnels masqués, qui ne soient pas de manipulation et de mise en oeuvre dangereuse et/ou délicate.

Un autre objectif de l'invention est de fournir de nouveaux polyisocyanates à groupements fonctionnels bloqués, qui soient économiques.

Un autre objectif de l'invention est de fournir de nouveaux polyisocyanates à groupements fonctionnels bloqués, donnant accès à des polymères (ou plutôt à de polycondensats), éventuellement réticulés, qui satisfassent au cahier des charges des applications.

Un autre objectif de l'invention est de fournir un procédé d'obtention de tels polyisocyanates bloqués.

Un autre objectif de l'invention est de fournir un procédé de préparation de polymères et/ou réticulats à partir desdits polyisocyanates bloqués.

Un autre but de la présente invention est de fournir des compositions comportant des isocyanates masqués des types précédents.

Un autre but de la présente invention est de fournir des compositions de poudre du type précité.

Un autre but de la présente invention est de fournir des émulsions aqueuses comportant des isocyanates de type ci-dessus.

Un autre but de la présente invention est de fournir des suspensions comportant des isocyanates du type ci-dessus.

Ces buts et d'autres qui apparaîtront par la suite sont atteints au moyen d'isocyanates, avantageusement de diisocyanates, de préférence de polyisocyanates, masqués. Ce composé est porteur d'une oxime où le carbone de la fonction oxime est porteur d'au moins une fonction électro-attractrice par le fait que les substituants du carbone de la fonction oxime, et notamment ladite fonction électro-attractrice, sont choisis de manière que la somme des constantes de Hammett σₚ soit au moins égale à.0,2, avantageusement à 0,25, de préférence à 0,3 et par le fait que lorsque la fonction électro-attractrice est une cétone, cette cétone n'est ni un acétyle, ni une cétone plus énolisable que le groupe acétyle.

Il convient de mentionner que d'une part l'usage des oximes de cétones usuelles sont déjà connues de l'homme du métier agent de masquage des isocyanate en particulier la méthyléthycétoxime (voir par exemple la demande de brevet japonaise publiée sous le N° 04236213 que d'autre part dans le document WO 94/15984 l'utilisation de la monoxime du diacétyle à été mentionnée dans une liste d'oximes susceptibles de masquer les isocyanates, et que auparavant le document chemical abstracts, vol.82, 1975, p96, abstract N°157929a avait exemplifié l'utilisation de la diacétyle monoxime comme agent masquant. Cependant aucun de ces documents n'enseignait que les oximes porteuses de fonction électro-attractrices étaient susceptibles de se libérer à une température plus basse que les oximes usuelles. En outre les oximes cétoniques citées dans les deux derniers documents, présentent des inconvénients qui seront développés ultérieurement.

Il est en outre souhaitable que les substituants du carbone de la fonction oxime soient choisis de manière que la somme des constantes de Hammett σₚ soit au plus égale à 1, avantageusement à 0,8, de préférence à 0,6.

Pour ce faire, au moins un des substituants du carbone de la fonction oxime peuvent être choisis parmi les fonctions électro-attractrices présentant une constante de Hammett σₚ au plus égale à 0,8 ; avantageusement à 0,6 ; de préférence à 0,5.

L'effet est surtout lié à la composante σᵢ et il est préférable que ces composantes inductive soient choisis de manière que la somme des constantes de Hammett σᵢ soit au moins égale à 0,1, avantageusement à 0,15. Il est en outre souhaitable que les substituants du carbone de la fonction oxime soient choisis de manière que la somme des constantes de Hammett σᵢ soit au plus égale à 0,5 ; avantageusement à 0,4.

Pour plus de détails sur les constantes de Hammett on peut se référer à la troisième édition du manuel écrit par monsieur le professeur Jerry March "advanced organic chemistry" (pages 242 à 250) et édité par John Wiley and sons.

Le plus souvent on choisira au moins un des substituants du carbone de la fonction oxime parmi ceux tels que carbonyles, y compris carboxylates, esters et amides, nitrile, sulfones, y compris sulfonates, phosphones (c'est-à-dire : -P(O)=), y compris phosphonates et phosphinates, voire nitro (mais le groupe nitro est souvent dangereux et trop électro-attracteur), les fonctions cétones, esters, amides (surtout N disubstitués), carbazide (protégé le cas échéant pour éviter qu'il ne porte d'autre hydrogène aussi réactif que celui de l'oxime, cf. infra), nitrile, onium, di(pseudo)halogéno (avantageusement chloro, de préférence fluoro), poly(pseudo)halogénométhyle (avantageusement chloro, de préférence fluoro), sulfone, sulfoxyde, les noyaux aromatiques globalement appauvris en électron.

Parmi les fonctions esters, on peut comprendre les orthoesters et parmi les fonctions cétones, les fonctions cétal et acétal.

Il est souhaitable qu'au moins un des deux, de préférence les deux atomes auxquels est rattaché le carbone de la fonction oxime soient des carbones.

Ainsi, au cours de l'étude qui a mené à la présente invention, il a été montré que les oximes possédant au moins un groupe électro-attracteur en alpha du carbone porteur de l'oxime tel que par exemple les oximes des alpha céto esters, lactones, amides ou des nitriles ou cétones constituent une nouvelle classe de groupes protecteurs thermolabiles des fonctions isocyanates. Les autres groupements électro-attracteurs dès lors qu'ils ne perturbent les diverses réactions de synthèse et de libération peuvent être utilisés.

La structure générale des oximes donnant naissance à des groupes protecteurs ou masquant selon la présente invention est la suivante : 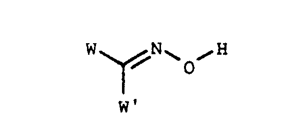 où au moins l'un des W et W' représente une fonction ou une fonction électro-attractrice ou un groupe électroattracteur, tel que décrit ci-dessus. l'autre des W et W' étant en général un radical hydrocarboné, notamment aryle (y compris alcoylaryle) ou alcoyle [dans la présente description **ALCO-*yle*** est pris dans son sens étymologique de reste hydrocarboné d'un **ALCO-*ol*** après ignorance de la fonction alcool (ou ol)].

Ainsi plus précisément, la structure générale préférées des oximes donnant naissance à des groupes protecteurs ou masquant selon la présente invention est la suivante. 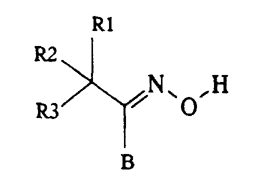 où B (correspondant à W) prend notamment les valeurs suivantes la flèche indique la place où aboutit la liaison entre le carbone de l'oxime et B

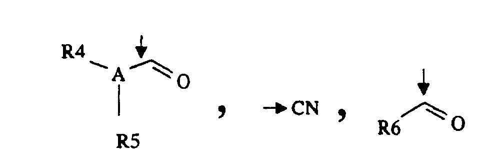

Avec R₁, R₂ , R₃ = H, ou alcoyle, y compris aralcoyle, ou aryle, y compris alcoylaryle ; (hétéro)cyclique ou non ;
Avec R₄ = alcoyle, ou aralcoyle, ou aryle, ou hétérocyclique et R₅ n'existe pas, quand A est Chalcogène ;
à condition que cela n'implique pas de réaction parasite, notamment quand A est carbone R₄ peut être hydrogène [c'est-à-dire qu'il ne soit pas d'hydrogène aussi ou plus acide que l'hydrogène mobile de la fonction oxime (hydrogène porté par l'oxygène de la fonction oxime voire infra)]

A représente un atome tétravalent porteur (carbone, voire de silicium) d'un substituant [H, ou alcoyle, y compris aralcoyle, ou aryle, y compris alcoylaryle ; (hétéro)cyclique ou non], d'azote (voire de phosphore), de chalcogène, de préférence léger (soufre et de préférence oxygène).

Avec R₄, R₅ = H, ou alcoyle, ou aralcoyle, ou aryle, ou hétérocyclique quand A est égal à azote.

Avec R₆ = alcoyle, y compris aralcoyle, ou aryle, ou hétérocyclique ; ainsi que mentionné ci-dessus R6 est avantageusement soumis à des contraintes notamment qu'il doit être tel que la cétone ne soit que difficilement énolisable.

Les R₁, R₂ , R₃ peuvent être reliés entre eux pour former un ou des cycles ;
Les R₄, R₅ peuvent être reliés entre eux pour former un cycle.

Font également partie de l'invention, les groupes protecteurs oximes qui possèdent deux groupes électro-attracteurs (où W est avantageusement B), semblables ou différents, en alpha du carbone portant l'oxime.

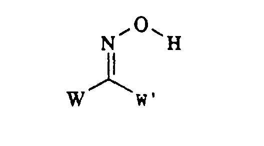

W et W', semblables ou différents, représentent les deux substituants du carbone de la fonction oxime, l'un d'entre eux au moins, ainsi que cela a été mentionné ci-dessus est un groupe électroattracteur. Il est souhaitable que W et W' ne soient pas simultanément aromatiques (c'est-à-dire qu'un cycle aromatique est directement lié au carbone de la fonction oxime). Lorsque W ou W' est aromatique, II est également souhaitable que la somme des constantes de Hammett σₚ des substituants du noyau aromatique (c'est-à-dire ledit noyau aromatique directement lié au carbone de la fonction oxime) soient inférieur à 0,5 de préférence à 0,4.

Sans que l'on puisse expliquer pourquoi, lorsque la fonction électroattractrice est une cétone, il est préférable que cette cétone ne soit pas facilement énolisable, c'est-à-dire qu'il convient d'éviter que lorsque la cétone jouant le rôle électroattracteur porte en alpha un hydrogène cet hydrogène soit facilement arrachable. La limite semble être la fonction acétyle. En d'autres termes, lorsque W et/ou W' représente une fonction cétone il est souhaitable que cette fonction ne soit pas plus facilement énolisable que le groupe acétyle CO-CH₃. ce dernier groupe n'est bien sûr pas lui-même l'un des préférés. Cela peut être codifié en indiquant que lorsque W ou W' est de la forme -CO-CH< les deux substituants (de droite) sont de préférence globalement électrodonneurs (rappelons que l'hydrogène est la référence et que sa constante de Hammett est par définition zéro et que les alcoyles stricto sensu sont réputés électrodonneurs), c'est à dire que la somme des constantes de Hammett σₚ des deux substituants (autres que le carbonyle) portés par CH soit négative.

Un inconvénient supplémentaire, mais non dirimant, des monoximes des gem dicétones (dicétones vicinales), surtout les symétriques, vient du fait que qu'il est difficile de mener une mono oximation, et que les gem dioximes ne sont pas non plus parmi les composés préférés [car elles sont conjuguées (et peuvent de ce fait être chromophore) et conduisent à des composés dont la réactivité n'est pas toujours aisée à maîtriser].

Eventuellement, W' et B, dont l'un au moins est attracteur, sont reliés entre eux et sont tels que l'oxime présente une structure cyclique, avec le cas échéant un ou plusieurs hétéroatomes pour former un hétérocycle dont la structure peut être symbolisée comme suit :

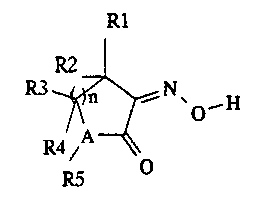

Avec R1, R2, R3, R4, R5 défini comme ci dessus et n pouvant prendre les valeurs entières entre 0 et 3.

On pourra moduler la réactivité et la température de déblocage de l'isocyanate protégé en jouant sur les groupes R1, R2, R3, A, R4, R5 et R6.

D'une manière générale, ces oximes utilisées comme agent de masquage des isocyanates ne doivent contenir, ni des fonctions qui seraient susceptibles de perturber la synthèse des isocyanates masqués, ni des fonctions qui seraient susceptibles de perturber l'aptitude à se conserver dans des conditions de stockage sans perte de qualité, ni des fonctions qui seraient susceptibles de perturber les réactions de condensation dans les conditions de température choisies.

En outre l'accessibilité à la fonction oxime doit être bonne dans la mesure du possible. Ainsi il est préférable qu'au moins un des deux carbones en position vicinale de celui de la fonction oxime, ne forme pas un groupe encombrant (en d'autres termes qu'il ne constitue avec ses substituants un carbone tertiaire).

Si l'on désire éviter la création d'autre liaisons que celle entre la fonction isocyanate et la fonction oxime, il convient d'éviter que ledit composé porteur de la fonction oxime possède d'autre fonction à hydrogène réactif [ou a tout le moins que cet hydrogène réactif le soit significativement moins que celui de la fonction oxime]. Ainsi on a alors, la condition que les substituants et notamment les fonctions électro-attractrices ne comportent pas d'hydrogène aussi ou plus acide que l'hydrogène mobile de la fonction oxime (hydrogène porté par l'oxygène de la fonction oxime).

La classification périodique des éléments utilisée dans la présente demande est celle du supplément au Bulletin de la Société Chimique de France, janvier 1966, n° 1.

Les éventuels substituants de l'oxime, peuvent être variés et être choisis parmi les substituants les plus variés pourvu qu'ils répondent à celles des conditions ci-dessus qui sont obligatoires, La dite oxime, agent de masquage des isocyanates selon l'invention, présente en général, par fonction oxime, au plus 50 atomes de carbone, avantageusement au plus 25, et de préférence au plus 15 atomes de carbone. Parmi les oximes utilisées au cours de la présente étude, celles qui étaient les plus pratiques à utiliser avait de 2 à 6 atomes de carbone par fonction oxime selon la présente invention.

Les oximes des aldéhydes donnent des résultats très nettement moins bons que ceux des oximes cétonique.

Les fonctions électro attractrice préférées sont celles qui comportent un carbonyle et dérive de la fonction acide. Ainsi les composés présentant l'oxime d'un dérivé d'un α cétoacide (notamment les dérivés des acides pyruviques) ne portant pas d'hydrogène réactif (voir infra) (tel que amide, imide, carboxylate, ester) sont particulièrement bien adaptés.

Ainsi que déjà mentionné ci-dessus, les isocyanates concernés peuvent être des mono-, di- voire poly-isocyanates. Ces dérivés peuvent contenir des structures de type isocyanurate, encore appelée trimère, des structures urétidine dione, encore appelée dimère, des structures biuret ou allophanate ou une combinaison de ce type de structures sur une seule molécule ou en mélange.

Les isocyanates monomères peuvent être :
aliphatiques, y compris cycloaliphatiques et aralaliphatiques, tels que :
   - les polyméthylènediisocyanates et notamment l'hexaméthylène diisocyanate ;
   - l'isophorone diisocyanate ;
   - les arylènedialcoylènediisocyanates (tel que OCN-CH₂-φ-CH₂-NCO) ;
ou encore aromatiques tels que le toluylène diisocyanate.

Les polyisocyanates préférés visés par la technique de masquage l'invention sont ceux dans lesquels au moins une, avantageusement deux, de préférence trois des conditions ci après sont remplies :
- au moins une, avantageusement deux, des fonctions NCO libre(s) ou à protéger sont reliées à un squelette hydrocarboné, par l'intermédiaire d'un carbone saturé (sp³) ;
- au moins un, avantageusement deux, desdits carbones saturés (sp³) est porteur d'au moins un, avantageusement deux, hydrogène(s),(en d'autre terme il a été trouvé que l'on obtenait de meilleurs résultats lorsque le carbone porteur de la fonction isocyanate était porteur d'un hydrogène, de préférence de deux hydrogènes) ; II est en outre même préférable que lesdits carbones saturés (sp³) soit au moins pour le tiers, avantageusement au moins pour la moitié, de préférence au moins pour les deux tiers, reliés audit squelette par un atome de carbone lui-même porteur d'au moins un hydrogène, plus préférentiellement deux ;
- tous les carbones par l'intermédiaire desquels les fonctions isocyanates sont reliées au squelette hydrocarboné, sont des carbones saturés (sp³), lesquels sont avantageusement en partie, de préférence en totalité, porteurs d'un hydrogène, de préférence de deux hydrogènes ; II est en outre même préférable que lesdits carbones saturés (sp³) soit au moins pour le tiers, avantageusement au moins pour la moitié, de préférence au moins pour les deux tiers, reliés audit squelette par un atome de carbone lui-même porteur d'au moins un hydrogène, plus préférentiellement deux ;

Lorsque les polyisocyanates sont relativement lourds c'est à dire qu'ils comportent au moins 4 fonctions isocyanates avantageusement masquées, ou bien lorsque l'on se trouve en face d'un mélanges de plusieurs composés porteurs de fonction(s) isocyanate(s), les premières conditions deviennent :
- au moins un tiers, avantageusement deux tiers, de préférence quatre cinquième des fonctions NCO libres ou à protéger sont reliées à un squelette hydrocarboné, par l'intermédiaire d'un carbone saturé (sp³).
- au moins un tiers, avantageusement deux tiers, de préférence quatre cinquième desdits carbones saturés (sp³) est porteur d'au moins un, avantageusement deux, hydrogène(s),(en d'autre terme il a été trouvé que l'on obtenait de meilleurs résultats lorsque le carbone porteur de la fonction isocyanate était porteur d'un hydrogène, de préférence de deux hydrogènes).
   - Il est en outre même préférable que lesdits carbones saturés (sp³) soit au moins pour le tiers, avantageusement au moins pour la moitié, de préférence au moins pour les deux tiers, reliés audit squelette par un atome de carbone lui-même porteur d'au moins un hydrogène, avantageusement deux ;
- sont particulièrement visé les mélanges pour lesquelles la (quasi) totalité des fonction isocyanate libres ou masquée réponde aux critères ci dessus .
En outre
- sont en particulier bien adaptés les mélanges ou les composés qui présentent au moins en partie (au moins le cinquième en masse par rapport aux fonctions NCO libres ou masquées, avantageusement un tiers, de préférence la moitié [la comparaison est aisée car les squelette en question sont, ou sont susceptibles d'être issus des fonctions isocyanates]) un squelette isocyanurique et/ou biuret (que ce squelette soit issu d'un seul ou de plusieurs monomères, voir ci-dessous) et plus précisément des structures de type isocyanurate, encore appelée trimère, des structures uretidinedione, encore appelée dimère, des structures biuret ou allophanate ou une combinaison de ce type de structures sur une seule molécule ou en mélange.

Ainsi l'invention porte notamment sur l'utilisation desdites oximes pour masquer tout ou partie des fonctions isocyanates d'un polyisocyanate, c'est à dire un composé présentant plusieurs fonctions isocyanates. Les fonctions non masquées selon la présente invention, peuvent être soit libres, soit masquées par d'autre groupes masquants. Les groupes masquants selon la présente invention sont particulièrement bien adaptés à la protection de ceux des groupements isocyanates qui sont aliphatiques et parmi ceux-là, ceux dont le carbone porteur de l'azote de la fonction isocyanate est saturé (sp³) et porteur d'un hydrogène, de préférence de deux hydrogènes.

En général les compositions d'isocyanates utilisées sont des mélanges de diverses molécules issues de polymérisations ou de polycondensation. auquel cas ce qui vient d'être expliqué sur ce qui est préféré ci dessus s'applique avec des valeurs fractionnaires et statistiques. Dans ce cas l'on peut indiquer qu'il est souhaitable que exprimé en fonction isocyanate (libre plus bloquée) et ne tenant pas compte du poids en agent bloquant, elle présente une teneur en fonction isocyanate comprise entre 1 et 35%, avantageusement 2 et 25%, de préférence entre 5 et 20%.

Lorsque l'on désire masquer une fonction isocyanate à caractère aromatique c'est à dire dont l'azote est lié à un carbone d'hybridation (sp²), il convient que les substituants du carbone porteur de la fonction oxime soit moins électro-attracteur, c'est à dire qui se situe dans la partie les moins électro-attractrice des fourchettes mentionnées ci dessus pour obtenir des valeurs qui répondent aux critères généraux ci dessous. Ainsi en général, pour les isocyanate aromatique la somme des constantes de Hammett σₚ est au plus égale à 0,45 ; avantageusement à 0,35 ; de préférence à 0,3.

Pour s'assurer, une bonne durée de vie au stockage, il est préférable de choisir des fonctions isocyanates masquées dont le test à l'octanol montre une "libération" à 80°C, avantageusement à 90°C, au plus égale à 90 %.

Pour les utilisations en solvant organique, en suspension ou en émulsion aqueuse, il est souhaitable de choisir des fonctions isocyanates masquées dont le test à l'octanol montre une "libération" à 150°C, avantageusement à 140°C, de préférence 130°C, au moins égale à 90 %.

Les isocyanates concernés peuvent être des mono-, di- voire polyisocyanates. Ces dérivés peuvent contenir des structures de type isocyanurate, encore appelée trimère, des structures uretidinedione, encore appelée dimère, des structures biuret ou allophanate ou une combinaison de ce type de structures sur une seule molécule ou en mélange.

La technique de préparation des isocyanates masqués à partir des isocyanates est une simple transposition des techniques utilisant les oximes usuelles telle que la Méthyléthylcétoxime (parfois désigné par son sigle anglo-saxon de MEKO) et qui conduit au trimère masqué MEKO lequel est vendu sous la marque Tolonate D2 et sert aux exemples comparatifs.

Rappelons que les oximes s'additionnent sur les fonctions isocyanates l'hydrogène allant sur l'azote cependant que l'oxygène se greffe sur le carbone du carbonyle :

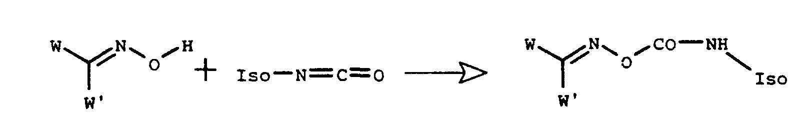

Iso représente le reste de la molécule de l'isocyanate, qui avantageusement comporte comme cela a été décrit auparavant d'autre(s) groupe(s) isocyanate(s), au moins un, de préférence au moins 2.

A titre d'enseignement par l'exemple on décrira ci-après en utilisant comme paradigme le masquage des composés désignés dans le domaine technique sous le nom de trimères de l'hexaméthylène diisocyanate tels ceux mis dans le commerce sous la marque déposée "Tolonates® HDT" ; ces composés sont issus de l'hexaméthylène diisocyanate par une trimérisation cyclique de trois fonctions isocyanates pour donner un cycle isocyanurique et en fait comprennent aussi divers autres polymères comme des pentamères (dans lequel un hexaméthylène diisocyanate voit ses deux fonctions isocyanates engagées dans un cycle isocyanurique) et tel que uretidinedione (dimère).

Ainsi dans les exemples où est décrit le masquage des trimères par les oximes des pyruvates de méthyle et d'éthyle, l'homme de métier pourra trouver des détails opératoires pour transposer les techniques utilisées avec des oximes des dialcoylcétones à celles selon l'invention et éviter ainsi des éventuels tests de routine.

Les synthèses de ces oximes est connue du spécialiste en la matière.

On peut notamment se reporter à la thèse monsieur Claude Mercier soutenue à Paris devant un jury présidé par le professeur Marc Julia.

L'un des nombreux intérêts des nouveaux polyisocyanates selon l'invention est qu'ils peuvent servir de base à la préparation de polymères et/ou de réticulats et être utilisés notamment comme un des constituants principaux de revêtements en tous genres, tels que vernis et peintures. Dans de telles utilisations, les qualités de dureté des polymères réticulables font partie de celles qui sont recherchées sur le plan technique et fonctionnelles.

Le susdit procédé de préparation de polymères comporte les étapes suivantes :
- mettre un polyisocyanate protégé selon l'invention (I) en présence d'un coréactif qui contient des dérivés présentant des hydrogènes réactifs sous forme d'alcool, de phénol, de Thiol, de certaines amines y compris les anilines; ces dérivés peuvent avoir des squelettes hydrocarbonés aliphatiques, alicycliques ou aromatiques, de préférence alcoyles, y compris cycloalcoyles et aralcoyles, aryles, linéaires ou branchés, substitués ou non ;(ces coréactifs, en général des polyols sont en eux même connus) [et constituer la couche (l'étaler) ou la forme selon l'application]
   et
- et chauffer le milieu réactionnel ainsi constitué à une température peu élevée par rapport au techniques usuelles, cela peut être soit une température élevée pendant un temps court ou une température faible pendant un temps long.
   Le premier cas correspond à la technique souvent désignée par le terme anglo-saxon de "coil coating" et correspond à une durée d'au plus 15 minutes à 180° et d'au plus 5 minute à 200°C. avec en plus une bonne résistance à la coloration (En général, jaunissement) pour les cas où il y aurait surcuisson.
   Dans l'autre extrême, il a une cuisson plus douce, En général, à une température d'au plus 160°C pendant une durée d'au plus deux heures, plus souvent d'au plus une heure.
   Avantageusement la température est au plus égale à 150°C, de préférence comprise entre 80 et 140° C et, plus préférentiellement encore, entre 110 et 130° C et ce, pour une durée inférieure cu égale à 15 h, de préférence à 10 h et, plus préférentiellement encore, à 8 h.

On peut prévoir d'inclure un solvant organique dans le milieu réactionnel. On peut également prévoir une suspension dans l'eau.

Ce solvant optionnel est, de préférence, peu polaire, c'est-à-dire dont la constante diélectrique n'est guère supérieure ou égale à 4 ou plus préférentiellement à 5.

Conformément à l'invention, les solvants peu polaires préférés sont, entre autres, les aromatiques tel que le benzène, le chlorobenzène (dichloro-1,2 benzène), le nitrobenzène, les cétones telles que la cyclohexanone, la méthyléthylcétone et l'acétone ; les esters d'alcoyle(s) léger et notamment les esters adipiques

Les dérivés entrant dans la composition du coréactif sont en général di-, oligo-, ou polyfonctionnel, peuvent être monomère ou issus de di-, d'oligo- ou polymérisation et sont mis en oeuvre pour la préparation de polyuréthannes éventuellement réticulés, leur choix sera dicté par les fonctionnalités attendues pour le polymère dans l'application finale et par leur réactivité.

Notamment lorsque l'on désire avoir des compositions "bi-composant" (c'est-à-dire contenant simultanément les deux réactifs : l'isocyanate ici au moins partiellement masqué selon l'invention et le composé à hydrogène réactif) stables préfère éviter d'utiliser des dérivés présentant des hydrogènes réactifs qui catalysent la libération de l'isocyanate masqué. Ainsi parmi les amines on préfère n'utiliser que celles qui ne catalyse pas la décomposition ou la transamidation des fonctions isocyanates masquées selon la présente invention.

Ces coréactifs sont en général bien connu de l'homme de métier.

L'invention concerne donc, également, des compositions de peintures comprenant pour addition successive ou simultanées :
- un polyisocyanate masqué selon l'invention ;
- un coréactif à hydrogène réactif tel que décrit supra ;
- d'éventuels catalyseurs en eux même connus pour les oximes et, notamment ceux à base d'étain ;
- éventuellement au moins un pigment ;
- éventuellement du bioxyde de titane ;
- éventuellement une phase aqueuse ;
- éventuellement un agent tensioactif pour maintenir en émulsion ou en suspension les composants constitutifs du mélange ;
- éventuellement un solvant organique ;
- éventuellement un déshydratant.

L'invention concerne aussi les peintures et vernis obtenues par l'utilisation de ces compositions, avec l'éventuelle libération selon le procédé ci-dessus.

### TEST A L'OCTANOL

### définitions

- température de "libération" (ou de "déblocage"):: c'est la température la plus faible à laquelle
- l'agent de masquage de l'isocyanate masqué est déplacé à hauteur de 9/10 (arrondi mathématique) par un monoalcool primaire (l'alcool primaire est en général l'octanol)..
- durée de vie au stockage :: Pour s'assurer, une bonne durée de vie au stockage, il est préférable de choisir des fonctions isocyanates masquées dont le test à l'octanol montre une "libération" à 80°C, avantageusement à 90°C, au plus égale à 90%.
- Avancement de la réaction :: On considère que la réaction est complète si elle est réalisée à plus de 90 %.

### mode opératoire

Dans un tube, type SCHOTT, avec agitation magnétique, on charge environ 5 mmol en équivalent NCO masqué protégé à évaluer.

On ajoute 2,5 à 3 ml de dichloro-1,2 benzène (solvant) l'équivalent d'octanol-1 (5 mmol, soit 0,61 g et eventuellement avec le catalyseur à tester avec le groupe masquant).

Le milieu réactionnel est ensuite porté à la température testée. On chauffe alors pendant 6 h à la température testée, de façon à débloquer et ainsi rendre réactives les fonctions isocyanates. La réaction terminée, le solvant est éliminé par distillation sous vide, et le résidu est analysé en RMN, Masse et infra rouge.

A partir de ces données, on évalue le pourcentage de fonction isocyanate masquée condensée avec l'octanol-1.

Les exemples non limitatifs suivants illustrent l'invention.

### Exemple N°1 : Synthèse de l'oxime du pyruvate de méthyle

Cette oxime a été préparée selon la procédure décrite dans le PRACTICAL ORGANIC CHEMISTRY, VOGEL 's, 5ème édition , édité chez LONGMAN SCIENTIFIC AND TECHNICAL, p 1259. Les pyruvatoximes ont également été décrites dans Synthetic Communications, 22 (22), 3263-3269 (1992).

Dans un ballon tricol, on charge successivement 5 g de pyruvate de méthyle (0.049 mole) (RN cas : 600-22-6), 50g d'éthanol, 5 g de chlorhydrate d'hydroxylamine ( 0.0719 mole) et 7.26 g de triéthylamine (0.0719 mole).Le milieu réactionnel est porté à 80°C. Après 1 h 30, le mélange réactionnel est concentré aux 3/4 à l'évaporateur rotatif. Après addition de 50 cc d'eau distillée, et refroidissement à 0°C, l'oxime de pyruvate de méthyle précipite. Le précipité est filtré.

Une autre procédure de traitement permet d'obtenir le produit. A la fin de la réaction le milieu réactionnel est concentré, le résidu est repris par 100 cc d'acétate d'éthyle. Le précipité de chlorhydrate de triéthylamine est éliminé par filtration. La phase organique est concentré et l'oxime formée cristallise.

La quantité d'oxime récupérée est de 2.72 g, qui est analysée par spectroscopie RMN, masse, et infra rouge. Les spectres correspondants sont présentés en annexe 1.

Il faut remarquer que faire la réaction dans l'éthanol comme solvant conduit à une réaction de transestérification de l'ester de l'oxime, c'est pourquoi l'on observe la présence d'ester éthylique et méthylique.

### Exemple N°2 : Synthèse de l'oxime du pyruvate d'éthyle (souvent désigné ci-après par son hypocoristique : "POME"))

Dans un tricol, on charge successivement, 25 g de pyruvate d'éthyle (0,215 mole), 70 cc d'éthanol, 25 g de chlorhydrate d'hydroxylamine ( 0,36 mole ) et 36,33 g de triéthylamine (0,36 mole). La réaction est mise au reflux pendant 2 heures. L'éthanol est éliminé par distillation sous vide, le résidu solide est alors repris par l'acétate d'éthyle. Le chlorhydrate de triéthylamine est filtré. La phase organique est concentré pour donner 4 g d'oxime.

La réaction n'a pas été optimisée.

### Exemple N°3 : synthèse de tolonates® masqués par des oximes voir ci-après

**Exemple N°4 :** Synthèse du Tolonate® HDT bloqué par l'oxime du pyruvate de méthyle.

Dans un tricol, on charge successivement 1,63 g de tolonate® HDT ( 8,54 10⁻³ mole de fonction isocyanate NCO), 30 cc de toluène, et 1,098g (8,54 10⁻³ mole) de BJ 349 ( oxime du pyruvate de méthyle). Après 3 heures de réaction à 50°C, l'analyse infra rouge indique que toutes les fonctions NCO ont été substituées. Le solvant est alors évaporé sous vide.

Ce produit est référencé XD2G

### Exemple N°5 : Synthèse du Tolonate® HDT bloqué par l'oxime du pyruvate d'éthyle.

Le mode opératoire est identique à celui indiqué précédemment

### Exemple N°6 : REACTION DE DEBLOCAGE DES Tolonates® BLOQUES

On a développé un test représentatif des propriétés de "libération" de la fonction isocyanate qui permet de comparer les températures de déblocage des Tolonates® bloqués.

### 6.1/ Définition du test à l'octanol

Dans un tube Schott de 50 cc, on introduit successivement, 0,517 g de Tolonate bloqué par du pyruvate de méthyle, 0,219 g d'octanol 1 (même concentration molaire que de fonction NCO protégée), et 5,02 g de dichlorobenzène. Le milieu réactionnel est ensuite porté à la température testée (dans le présent exemple à 100°C) pendant 6 heures. La réaction terminée, le solvant est éliminé par distillation sous vide, et le résidu est analysé en RMN, Masse et infra rouge.

### 6.2/ Résultats

### 6.2.1 Cas du Tolonate® HDT bloqué oxime du pyruvate de méthyle.

Le pourcentage de déblocage et formation de carbamate d'octyle attendu est de 80% alors que dans les mêmes conditions un Tolonate® bloqué par la méthyle éthyle cétoxime n'est pas déprotégé et ne donne pas de carbamate d'octyle.

### 6.2.2 Cas du Tolonate® HDT bloqué oxime du pyruvate d'éthyle.

Le pourcentage de déblocage et formation de carbamate d'octyle attendu est de 47 % alors que dans les mêmes conditions un Tolonate® bloqué par la méthyléthykétoxime n'est pas déprotégé et ne donne pas de carbamate d'octyle.

Le test donne une indication très favorable sur l'aptitude du produit masqué à être stocké pendant longtemps.

### CONCLUSION des essais

II a été montré que les oximes appauvries en électron et notamment celles des alpha cétoesters, amides ou nitriles peuvent être de bons groupes protecteurs des fonctions isocyanates thermorégénérables à basse température. Les isocyanates bloqués par de tels groupes conduisent à une réaction de carbamatation par réaction avec l'octanol-1, à basse température, à partir de 100°C, ce qui constitue un avantage particulièrement important dans la nouvelle génération de peintures qualifiée de "monocomposant".

### Exemple N°7 : Essais comparatifs de préparation de peintures et vernis. TOLONATE XD2G (HDT bloqué POME) Evaluation par rapport au TOLONATE D2 (HDT bloqué MEKO)

### I. Etude en vernis :

Le polyol utilisé est une résine acrylique de chez Henkel, G-Cure 105P70 (taux d'OH = 3.06% par rapport au sec et extrait sec = 70.2%) : cf. fiche technique P-303 de septembre 1993.

Le Tolonate D2 est livré à 74% d'extrait sec dans le Solvesso 100, avec un taux de NCO de 11.2% sur le produit tel quel.

Le XD2G est à 75% d'extrait sec dans le Solvesso 100, avec un taux de NCO sur la forme de livraison de 13.08%.

Des vernis à NCO/OH = 1.08 et un extrait sec de 50% sont préparés avec 0.05% de DBTL par rapport au sec et sans catalyseur :

| | Sans catalyseur | Avec DBTL | Sans catalyseur | Avec DBTL |
|---|---|---|---|---|
| G-Cure 105 P 70 | 22,78 | 22,78 | 23,86 | 23,86 |
| Isocyanate | 11,62 | 11,62 | 10,43 | 10,43 |
| Solvant* | 15,60 | 14,35 | 15,71 | 14,46 |
| DBTL (solution à 1%) | - | 1,25 | - | 1,25 |

| | | | | |
|---|---|---|---|---|
| * : Le solvant est constitué d'un mélange de 57% d'EEP (éthyl 3 éthoxy propionate), de 40% de Solvesso 100 et de 3% de Rhodiasolve RPDE. | | | | |

Après 15 minutes de mélange puis 15 minutes de débullage, des films sont appliqués au tire-film automatique (environ 50µm secs) sur des plaques de verre et passés à l'étuve à différents temps et différentes températures.

### 1,1/ Température de déblocage :

On évalue la température de déblocage par la mesure de la dureté Persoz et par le test de la résistance à la goutte de MEK (méthyl éthyl cétone).

Dureté Persoz (en secondes) :

| **Cuisson** | **Témoins (avec D2)** | | **Formules avec XD2G** | |
|---|---|---|---|---|
| | Sans catalyseur | Avec DBTL | Sans catalyseur | Avec DBTL |
| 30 min à 140°C | 156" | 265" | 349" | 400" |
| 20 min à 150°C | 136" | 244" | 342" | 403" |
| 30 min à 150°C | 253" | 384" | 396" | 401" |
| 40 min à 150°C | 324" | 399" | 400" | 397" |
| 30 min à 160°C | 383" | 397" | 401" | 396" |

Avec cette seule information, on peut dire que le TOLONATE D2 est réticulé après 30 min à 160°C sans catalyseur et après 30 min à 150°C avec 0,05% de DBTL; et que le XD2G l'est après 30 min à 150°C sans catalyseur et après 30 min à 140°C avec DBTL.

On peut affiner ces premières estimations par la résistance du film à la MEK :

| **Cuisson** | **Témoins (avec D2)** | | **Formules avec XD2G** | |
|---|---|---|---|---|
| | Sans catalyseur | Avec DBTL | Sans catalyseur | Avec DBTL |
| 30 min à 140°C | 3 | 2 | 2 | 1 |
| 20 min à 150°C | 3 | 1 | 2 | 1 |
| 30 min à 150°C | 2 | 1 | 1 | 1-0 |
| 40 min à 150°C | 2-1 | 0-1 | 1 | 1-0 |
| 30 min à 160°C | 1 | 1 | 1 | 1-0 |

La cctation va de 5 (film totalement attaqué) à 0 (aucune trace visible).

En utilisant les deux tableaux ci-dessus, on peut donc donner les résultats suivants :

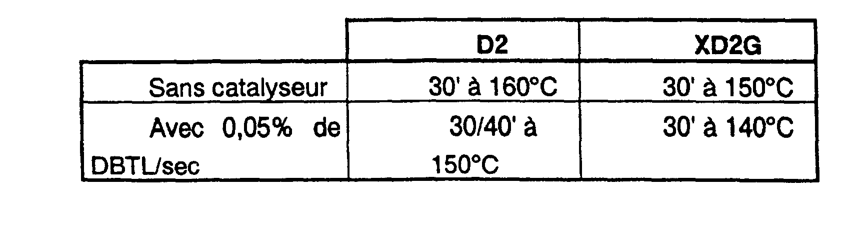

Ces valeurs ont été confirmées par une étude postérieure faite non plus en utilisant une étuve ventilée traditionnelle et en faisant des cuissons à différents temps et températures mais sur un four à gradient de la société BYK Gardner.

### 1.2/ Jaunissement à la surcuisson :

Le jaunissement est mesuré au spectrocolorimètre MINOLTA par l'indice de jaune, sur des films étuvés à différents temps à 200°C.

| **Cuisson** | **Témoins (avec D2)** | | **Formules avec XD2G** | |
|---|---|---|---|---|
| | Sans catalyseur | Avec DBTL | Sans catalyseur | Avec DBTL |
| 30 min à 150°C | 3,21 | 3,26 | 3,45 | 3,44 |
| 15 min à 200°C | 4,39 | 4,42 | 4,18 | 4,51 |
| 30 min à 200°C | 7,47 | 8,65 | 7,69 | 7,46 |
| 60 min à 200°C | 13,64 | 18,13 | 13,28 | 12,90 |

Le TOLONATE D2 et le XD2G présentent des indices de jaune comparables lorsque les films ne contiennent pas de catalyseur. Or, en pratique, les formulateurs en utilisent dans la majorité des cas. En présence de DBTL, le XD2G a tendance à nettement moins jaunir à la surcuisson que le D2.

### II. Etude en peinture :

Une formule de peinture blanche représentative du coil-coating, inspirée d'une formule d'orientation de DSM Resins (cf. fiche 92/12-F12) a été préparée :

| | **Produit** | **masse** | **Fournisseur** | **Désignation** |
|---|---|---|---|---|
| 1 | Uralac SN 830 S2-60 | 181,63 | DSM Resins | résine polyester |
| 2 | Solvesso 150 | 39,60 | ESSO | solvant |
| 3 | Dowanol PnB | 13,20 | Dow Chemicals | solvant |
| 4 | Disperbyk 160 + xylène (50/50) | 9,51 | BYK | dispersant |
| 5 | Rhoditan RL 60 Broyage puis ajout | 327,35 | Rhône-Poulenc | pigment TiO₂ |
| 6 | Uralac SN 830 S2-60 | 285,10 | DSM Resins | résine polyester |
| 7 | DBTL à 50% dans Solvesso 100 | 1,37 | Adriss | catalyseur |
| 8 | Solvesso 150 | 87,51 | ESSO | solvant |
| 9 | Dowanol PnB | 29,17 | Dow Chemicals | solvant |
| 10 | BYK 358 | 4,44 | BYK | agent de tension |
| 11 | Rhodiasolve RPDE | 21,12 | Rhône-Poulenc | agent d'étalement |
| | | 1000,00 | | |

L'Uralac SN 830 est un polyester saturé à faible teneu: en OH (environ 1% par rapport au sec), à 60% d'extrait sec dans le Solvesso 150, très souple (TG = 26°C), de poids moléculaire relativement faible (Mn = 4500), fabriqué par DSM Resins (cf. fiche technique 92/12-F12).

### Mise en oeuvre :

Disperser sous agitation les ingrédients 1 à 5 dans l'ordre. Après incorporation de l'oxyde de titane, maintenir une forte agitation (environ 2000 tr/min) pendant une dizaine de minutes. Un broyage à l'aide de mico-billes de verre est ensuite effectué jusqu'à l'obtention d'une finesse supérieure à 8 à la jauge de North. Le complément (ingrédients 6 à 11) est ensuite fait dans l'ordre, sous agitation. L'isocyanate est enfin ajouté dans la base blanche afin d'avoir NCO/OH = 1,05 :

| | **Témoin (avec D2)** | **Peinture avec XD2G** |
|---|---|---|
| Base blanche | 1000,00 g | 1000,00 g |
| Isocyanate | 67,65 g | 57,93 g |

Après 15 min de mélange et 15 min de débullage, des films sont appliqués à l'aide d'un applicateur sur des tôles d'acier (environ 50 µm secs).

Les TOLONATE D2 et XD2G ont été testés en surcuisson dans cette peinture dans des conditions du prélaqué (passage très rapide dans étuve à 300°C). L'indice de jaune a ensuite été mesuré, sachant que le témoin est réticulé après 50" alors que la peinture avec le XD2G l'est après 40":

| **Cuisson** | **Témoin (avec D2)** | **Peinture avec XD2G** |
|---|---|---|
| 40" dans étuve à 300°C | 2,48 | 1,40 |
| 50" dans étuve à 300°C | 3,22 | 1,89 |
| 60" dans étuve à 300°C | 4,21 | 3,03 |
| 75" dans étuve à 300°C | 5,89 | 4,04 |
| 90" dans étuve à 300°C | 7,92 | 6,01 |

C'est donc le XD2G qui donne les meilleurs résultats (plus faible jaunissement à la surcuisson).

## Revendications

1. Isocyanate, avantageusement diisocyanate, de préférence polyisocyanate, **caractérisé par le fait qu'**il est porteur d'au moins une fonction isocyanate masquée susceptible d'être obtenue par l'action sur un isocyanate d'un composé porteur d'une oxime où le carbone de la fonction oxime est porteur d'au moins une fonction électro-attractrice **par le fait que** les substituants du carbone de la fonction oxime, et notamment ladite fonction électro-attractrice, sont choisis de manière que la somme des constantes de Hammett σₚ soit au moins égale à.0,2, avantageusement à 0,25, de préférence à 0,3 et **par le fait que** lorsque la fonction électro-attractrice est une cétone, cette cétone n'est ni un acétyle, ni une cétone plus énolisable que le groupe acétyle.

2. Isocyanate selon la revendication 1, **caractérisé par le fait que** les substituants du carbone de la fonction oxime sont choisis de manière que la somme des constantes de Hammett σₚ soit au plus égale à 1, avantageusement à 0,8, de préférence à 0,6.

3. Isocyanate selon les revendications 1 et 2, **caractérisé par le fait que** l'un au moins des substituants du carbone de la fonction oxime est choisi parmi les fonctions électro-attractrices présentant une constante de Hammett σₚ, au plus égale à 0,8, avantageusement à 0,6, de préférence à 0,5.

4. Isocyanate selon les revendications 1 à 3, **caractérisé par le fait que** au moins un des substituants du carbone de la fonction oxime est choisi parmi les fonctions cétones, esters, amides, nitrile, onium, di(pseudo)halogéno (avantageusement chloro, de préférence fluoro), poly(pseudo)halogénométhyle, sulfone, sulfoxyde, les noyaux aromatiques globalement appauvris en électron.

5. Isocyanate selon les revendications 1 à 4, **caractérisé par le fait que** les deux atomes auxquels est rattaché le carbone de la fonction oxime sont des carbones.

6. Isocyanate selon les revendications 1 à 5, **caractérisé par le fait que** ladite oxime est l'oxime d'un dérivé d'un α-cétoacide.

7. Isocyanate selon la revendication 6, **caractérisé par le fait que** ledit α-cétoacide est l'acide pyruvique.

8. Isocyanate selon les revendications 1 à 7, **caractérisé par le fait que** ladite oxime est l'oxime d'un ester d'un α-cétoacide, avantageusement de l'acide pyruvique.

9. isocyanate selon la revendication 8, **caractérisé par le fait que** ladite oxime est l'oxime du pyruvate de méthyle.

10. Isocyanate selon la revendication 8, **caractérisé par le fait que** ladite oxime est l'oxime du pyruvate d'éthyle.

11. Composition comportant au moins un composé porteur d'au moins deux fonctions isocyanates, avantageusement comportant au moins un composé porteur d'au moins trois fonctions isocyanates, au moins partiellement masquées, **caractérisée par le fait que** ledit composé est porteur d'au moins une fonction isocyanate masquée susceptible d'être obtenue par l'action sur un isocyanate d'un composé porteur d'une oxime où le carbone de la fonction oxime est porteur d'au moins une fonction électro-attractrice et **par le fait que** lorsque la fonction électro-attractrice est une cétone, cette cétone n'est ni un acétyle, ni une cétone plus énolisable que le groupe acétyle.

12. Composition selon la revendication 11, **caractérisée par le fait qu'**elle comporte en outre une phase aqueuse.

13. Composition comportant au moins un composé porteur d'au moins deux fonctions isocyanates, avantageusement comportant au moins un composé porteur d'au moins trois fonctions isocyanates, au moins partiellement masquées, **caractérisée par le fait que** ledit composé est porteur d'au moins une fonction isocyanate masquée susceptible d'être obtenue par l'action sur un isocyanate d'un composé porteur d'une oxime où le carbone de la fonction oxime est porteur d'au moins une fonction électro-attractrice et **par le fait qu'**elle comporte en outre une phase aqueuse.

14. Composition selon l'une des revendications 11 à 13, **caractérisée par le fait que** ledit composé porteur d'au moins une fonction isocyanate masquée susceptible d'être obtenue par l'action sur un isocyanate d'un composé porteur d'une oxime est un isocyanate selon les revendications 1 à 10.

15. Composition selon l'une des revendications 11 à 14, comportant un mélange de composés porteurs de fonction(s) isocyanate(s) éventuellement masquée(s), **caractérisée par le fait que** ledit mélange présente une fonctionnalité [nombre de fonction(s) isocyanate(s), masquée(s) ou non, par molécule en contenant] moyenne supérieure à 2, avantageusement au moins égale à 2,1 et au plus égale à environ 5, avantageusement à 4.

16. Composition selon la revendication 15, **caractérisée par le fait que** ledit mélange présente une fonctionnalité moyenne au moins égale à 2,4.

17. Composition selon les revendications 16 et 15, **caractérisée par le fait que** ledit mélange présente une fonctionnalité moyenne au plus égale à 3,7.

18. Composition selon les revendications 15 à 17, **caractérisée par le fait que** ledit mélange remplit au moins une, avantageusement au moins deux, de préférence au moins trois des conditions ci après :
• au moins un tiers, avantageusement deux tiers, de préférence quatre cinquième des fonctions NCO libres ou masquées sont reliées à un squelette hydrocarboné, par l'intermédiaire d'un carbone saturé (sp³) ;
• au moins un tiers, avantageusement deux tiers, de préférence quatre cinquième desdits carbones saturés (sp³) est porteur d'au moins un, avantageusement deux hydrogènes ;
• lesdits carbones saturés (sp³) sont au moins pour le tiers, avantageusement au moins pour la moitié, de préférence au moins pour les deux tiers, reliés audit squelette par un atome de carbone lui-même porteur d'au moins un hydrogène, avantageusement deux.

19. Composition selon les revendications 11 à 18, **caractérisée par le fait que** lesdits composés porteurs de fonctions isocyanates présentent au moins en partie des structures de type isocyanurate, des structures uretidinedione, des structures biuret ou allophanate ou une combinaison de ce type de structures

20. Composition selon la revendication 19, **caractérisée par le fait que** lesdites structures sont issues de monomères aliphatiques.

21. Composition selon la revendication 20, **caractérisée par le fait que** lesdits monomères aliphatiques sont des polyméthylènediisocyanates.

22. Composition selon les revendications 11 à 21, utile pour le revêtement, **caractérisée par le fait qu'**elle comporte en outre, pour addition successive ou simultanée, un co-réactif à hydrogène réactif.

23. Composition selon les revendications 11 à 22, **caractérisée par le fait qu'**elle comporte en outre au moins un catalyseur de démasquage en lui-même connu pour les isocyanates masqués par des oximes.

24. Utilisation des compositions selon les revendications 11 à 23, pour la réticulation d'un revêtement

25. Utilisation des compositions selon la revendication 24, pour la réticulation d'un revêtement selon le procédé dit «coil coating».

26. Utilisation des compositions selon la revendication 24, pour la réticulation d'un revêtement à une température comprise entre 110°C et 130°C

27. Procédé de préparation de polymères, **caractérisé par le fait qu'**il comporte les étapes suivantes :
□ mettre un polyisocyanate protégé selon l'une des revendications 1 à 10 (I) en présence d'un co-réactif qui contient des dérivés présentant des hydrogènes réactifs ; et
□ chauffer le milieu réactionnel ainsi constitué selon la technique du coil coating ou à une température comprise entre 110°C et 130°C.

## Claims

1. Isocyanate, advantageously diisocyanate, preferably polyisocyanate, **characterized in that** it carries at least one masked isocyanate functional group capable of being obtained by the action on an isocyanate of a compound carrying an oxime where the carbon of the oxime functional group carries at least one electron-withdrawing functional group, **in that** the substituents of the carbon of the oxime functional group, and especially the said electron-withdrawing functional group, are chosen so that the sum of the Hammett constants σₚ is at least equal to 0.2, advantageously 0.25, preferably 0.3, and **in that** when the electron-withdrawing functional group is a ketone, this ketone is neither an acetyl nor a ketone more enolizable than the acetyl group.

2. Isocyanate according to Claim 1, **characterized in that** the substituents of the carbon of the oxime functional group are chosen so that the sum of the Hammett constants σₚ is at most equal to 1, advantageously 0.8, preferably 0.6.

3. Isocyanate according to Claims 1 and 2, **characterized in that** at least one of the substituents of the carbon of the oxime functional group is chosen from the electron-withdrawing functional groups having a Hammett constant σₚ at most equal to 0.8, advantageously 0.6, preferably 0.5.

4. Isocyanate according to Claims 1 to 3, **characterized in that** at least one of the substituents of the carbon of the oxime functional group is chosen from ketone, ester, amide, nitrile, onium, di(pseudo)halo (advantageously chloro, preferably fluoro), poly(pseudo)halomethyl, sulphone and sulphoxide functional groups and aromatic nuclei which are electron-depleted overall.

5. Isocyanate according to Claims 1 to 4, **characterized in that** the two atoms to which the carbon of the oxime functional group is attached are carbons.

6. Isocyanate according to Claims 1 to 5, **characterized in that** the said oxime is the oxime of a derivative of an α-ketoacid.

7. Isocyanate according to Claim 6, **characterized in that** the said α-ketoacid is pyruvic acid.

8. Isocyanate according to Claims 1 to 7, **characterized in that** the said oxime is the oxime of an ester of an α-ketoacid, advantageously of pyruvic acid.

9. Isocyanate according to Claim 8, **characterized in that** the said oxime is the oxime of methyl pyruvate.

10. Isocyanate according to Claim 8, **characterized in that** the said oxime is the oxime of ethyl pyruvate,

11. Composition comprising at least one compound carrying at least two isocyanate functional groups, advantageously comprising at least one compound carrying at least three isocyanate functional groups, which are at least partially masked, **characterized in that** the said compound carries at least one masked isocyanate functional group capable of being obtained by the action on an isocyanate of a compound carrying an oxime where the carbon of the oxime functional group carries at least one electron-withdrawing functional group, and **in that** when the electron-withdrawing functional group is a ketone, this ketone is neither an acetyl nor a ketone more enolizable than the acetyl group.

12. Composition according to Claim 11, **characterized in that** it additionally comprises an aqueous phase.

13. Composition comprising at least one compound carrying at least two isocyanate functional groups, advantageously comprising at least one compound carrying at least three isocyanate functional groups, which are at least partially masked, **characterized in that** the said compound carries at least one masked isocyanate functional group capable of being obtained by the action on an isocyanate of a compound carrying an oxime where the carbon of the oxime functional group carries at least one electron-withdrawing functional group and **in that** it furthermore includes an aqueous phase.

14. Composition according to one of Claims 11 to 13, **characterized in that** the' said compound carrying at least one masked isocyanate functional group capable of being obtained -by the action on an isocyanate of a compound carrying an oxime is an isocyanate according to Claims 1 to 10.

15. Composition according to one of Claims 11 to 14, comprising a mixture of compounds carrying optionally masked isocyanate functional group(s), **characterized in that** the said mixture has a mean functionality [number of isocyanate functional group(s), masked or otherwise, per molecule containing it(them)] greater than 2, advantageously at least equal to 2.1 and at most equal to approximately 5, advantageously to 4.

16. Composition according to Claim 15, **characterized in that** the said mixture has a mean functionality at least equal to 2.4.

17. Composition according to Claims 16 and 15, **characterized in that** the said mixture has a mean functionality at most equal to 3.7.

18. Composition according to Claims 15 to 17, **characterized in that** the said mixture meets at least one, advantageously at least two, preferably at least three of the following conditions:
• at least one third, advantageously two thirds, preferably four fifths, of the free or masked NCO functional groups are linked to a hydrocarbon backbone via a saturated (sp³) carbon;
• at least one third, advantageously two thirds, preferably four fifths, of the said saturated (sp³) carbons carries at least one, advantageously two, hydrogen(s);
• at least one third, advantageously at least half, preferably at least two thirds, of the said saturated (sp³) carbons are linked to the said backbone by a carbon atom itself carrying at least one hydrogen, advantageously two.

19. Composition according to Claims 11 to 18, **characterized in that** the said compounds carrying isocyanate functional groups exhibit, at least in part, isocyanurate-type structures, uretidinedione structures, biuret or allophanate structures or a combination of these types of structures.

20. Composition according to Claim 19, **characterized in that** the said structures come from aliphatic monomers.

21. Composition according to Claim 20, **characterized in that** the said aliphatic monomers are polymethylene diisocyanates.

22. Composition according to Claims 11 to 21, which can be used for coating, **characterized in that** it additionally comprises for successive or simultaneous addition a coreactant containing reactive hydrogen.

23. Composition according to Claims 11 to 22, **characterized in that** it additionally comprises at least one demasking catalyst itself known for isocyanates masked by oximes.

24. Use of the compositions according to Claims 11 to 23, for the crosslinking of a coating.

25. Use of the compositions according to Claim 24, for the crosslinking of a coating using the process referred to as coil coating.

26. Use of the compositions according to Claim 24, for the crosslinking of a coating at a temperature of between 110°C and 130°C.

27. Process for the preparation of polymers, **characterized in that** it comprises the following stages:
□ bringing a polyisocyanate protected according to one of Claims 1 to 10 into contact with a coreactant which contains derivatives which have reactive hydrogens;
and
□ heating the reaction mixture thus formed according to the coil coating technique or to a temperature of between 110°C and 130°C.

## Patentansprüche

1. Isocyanat, vorteilhafterweise Diisocyanat, vorzugsweise Polyisocyanat, **dadurch gekennzeichnet, dass** es wenigstens eine maskierte Isocyanatfunktion trägt, die durch Einwirkung einer Verbindung, die ein Oxim trägt, worin der Kohlenstoff der Oximfunktion wenigstens eine elektronenziehende Funktion trägt, auf ein Isocyanat erhalten werden kann, dadurch, dass die Substituenten des Kohlenstoffs der Oximfunktion und insbesondere besagte elektronenziehende Punktion derart ausgewählt sind, dass die Summe der Hammett-Konstanten σₚ wenigstens gleich 0,2, vorteilhafterweise wenigstens 0,25, vorzugsweise wenigstens 0,3 ist, und dadurch, dass, wenn die elektronenziehende Funktion ein Keton ist, dieses Keton weder ein Acetyl noch ein stärker als die Acetylgruppe enolisierbares Keton ist.

2. lsocyanat gemäß Anspruch 1, **dadurch gekennzeichnet, dass** die Substituenten des Kohlenstoffs der Oximfunktion derart ausgewählt sind, dass die Summe der Hammett-Konstanten σₚ höchstens gleich 1, vorteilhafterweise höchstens 0,8, vorzugsweise höchstens 0,6 ist.

3. Isocyanat gemäß den Ansprüchen 1 und 2, **dadurch gekennzeichnet, dass** wenigstens einer der Substituenten des Kohlenstoffs der Oximfunktion unter den elektronenziehenden Funktionen ausgewählt ist, die eine Hammett-Konstante σₚ von höchstens gleich 0,8, vorteilhafterweise höchstens 0,6, vorzugsweise höchstens 0,5 aufweisen.

4. Isocyanat gemäß den Ansprüchen 1 bis 3, **dadurch gekennzeichnet, dass** wenigstens einer der Substituenten des Kohlenstoffs der Oximfunktion ausgewählt ist unter den Keton-, Ester-, Amid-, Nitril-, Onium-, Di(pseudo)halogen- (vorteilhafterweise Chlor-, vorzugsweise Fluor-), Poly(pseudo)halogenmethyl-, Suifon-, Sutfoxidfunktionen, den aromatischen, insgesamt an Elektronen verarmten Ringen.

5. lsocyanat gemäß den Ansprüchen 1 bis 4, **dadurch gekennzeichnet, dass** die beiden Atome, an die der Kohlenstoff der Oximfunktion gebunden ist, Kohlenstoffe sind.

6. Isocyanat gemäß den Ansprüchen 1 bis 5, **dadurch gekennzeichnet, dass** besagtes Oxim das Oxim eines Derivats einer α-Ketosäure ist.

7. lsocyanat gemäß Anspruch 6, **dadurch gekennzeichnet, dass** besagte α-Ketosäure Brenztraubensäure ist.

8. lsocyanat gemäß den Ansprüchen 1 bis 7, **dadurch gekennzeichnet, dass** besagtes Oxim das Oxim eines Esters einer α-Ketosäure, vorteilhafterweise von Brenztraubensäure ist.

9. Isocyanat gemäß Anspruch 8, **dadurch gekennzeichnet, dass** besagtes Oxim das Oxim von Methylpyruvat ist.

10. lsocyanat gemäß Anspruch 8, **dadurch gekennzeichnet, dass** besagtes Oxim das Oxim von Ethylpyruvat ist.

11. Zusammensetzung umfassend wenigstens eine Verbindung, die wenigstens zwei Isocyanatfunktionen trägt, vorteilhafterweise umfassend wenigstens eine Verbindung, die wenigstens drei Isocyanatfunktionen trägt, die wenigstens teilweise maskiert sind, **dadurch gekennzeichnet, dass** besagte Verbindung wenigstens eine maskierte Isocyanatfunktion trägt, die durch Einwirkung einer Verbindung, die ein Oxim trägt, worin der Kohlenstoff der Oximfunktion wenigstens eine elektronenziehende Funktion trägt, auf ein Isocyanat erhalten werden kann, und dadurch, dass, wenn die elektronenziehende Funktion ein Keton ist, dieses Keton weder ein Acetyl noch ein stärker als die Acetylgruppe enolisierbares Keton ist

12. Zusammensetzung gemäß Anspruch 11, **dadurch gekennzeichnet, dass** sie außerdem eine wässrige Phase umfasst.

13. Zusammensetzung umfassend wenigstens eine Verbindung, die wenigstens zwei Isocyanatfunktionen trägt, vorteilhafterweise umfassend wenigstens eine Verbindung, die wenigstens drei Isocyanatfunktionen trägt, die wenigstens teilweise maskiert sind, **dadurch gekennzeichnet, dass** besagte Verbindung wenigstens eine maskierte Isocyanatfunktion trägt, die durch Einwirkung einer Verbindung, die ein Oxim trägt, worin der Kohlenstoff der Oximfunktion wenigstens eine elektronenziehende Funktion trägt, auf ein Isocyanat erhalten werden kann, und dadurch, dass sie außerdem eine wässrige Phase umfasst.

14. Zusammensetzung gemäß einem der Ansprüche 11 bis 13, **dadurch gekennzeichnet, dass** besagte Verbindung, die wenigstens eine maskierte Isocyanatfunktion trägt, die durch die Einwirkung einer Verbindung, die ein Oxim trägt, auf ein lsocyanat erhalten werden kann, ein Isocyanat gemäß den Ansprüchen 1 bis 10 ist.

15. Zusammensetzung gemäß einem der Ansprüche 11 bis 14 umfassend ein Gemisch von Verbindungen, die gegebenenfalls maskierte Isocyanatfunktion (en) tragen, **dadurch gekennzeichnet, dass** besagtes Gemisch eine mittlere Funktionalität [Anzahl an maskierter(en) oder nicht maskierter(en) lsocyanatfunktion(en) pro Molekül, das davon enthält] größer als 2, vorteilhafterweise von wenigstens gleich 2,1 und von höchstens gleich etwa 5, vorteilhafterweise höchstens 4 aufweist.

16. Zusammensetzung gemäß Anspruch 15, **dadurch gekennzeichnet, dass** besagtes Gemisch eine mittlere Funktionalität von wenigstens gleich 2,4 aufweist.

17. Zusammensetzung gemäß den Ansprüchen 16 und 15, **dadurch gekennzeichnet, dass** besagtes Gemisch eine mittlere Funktionalität von höchstens gleich 3,7 aufweist.

18. Zusammensetzung gemäß den Ansprüchen 15 bis 17, **dadurch gekennzeichnet, dass** besagtes Gemisch wenigstens eine, vorteilhafterweise wenigstens zwei, vorzugsweise wenigstens drei der folgenden Bedingungen erfüllt:
• wenigstens ein Drittel, vorteilhafterweise zwei Drittel, vorzugsweise vier Fünftel der freien oder maskierten NCO-Funktionen sind über einen gesättigten (sp³) Kohlenstoff an ein Kohlenwasserstoffgerüst gebunden;
• wenigstens ein Drittel, vorteilhafterweise zwei Drittel, vorzugsweise vier Fünftel besagter gesättigter (sp³) Kohlenstoffe trägt wenigstens einen, vorteithafterweise zwei Wasserstoffe;
• besagte gesättigte (sp³) Kohlenstoffe sind zu wenigstens einem Drittel, vorteilhafterweise zu wenigstens der Hälfte, vorzugsweise zu wenigstens zwei Dritteln an besagtes Gerüst über ein Kohlenstoffatom, das selbst wenigstens einen, vorteilhafterweise zwei Wasserstoffe trägt, gebunden.

19. Zusammensetzung gemäß den Ansprüchen 11 bis 18, **dadurch gekennzeichnet, dass** besagte Verbindungen, die Isocyanatfunktionen tragen, wenigstens teilweise Strukturen vom Isocyanurat-Typ, Uretidindion-Strukturen, Biuret- oder Allophanat-Strukturen oder eine Kombination dieses Typs von Strukturen aufweisen.

20. Zusammensetzung gemäß Anspruch 19, **dadurch gekennzeichnet, dass** besagte Strukturen aus aliphatischen Monomeren hervorgegangen sind.

21. Zusammensetzung gemäß Anspruch 20, **dadurch gekennzeichnet, dass** besagte aliphatische Monomere Polymethylendiisocyanate sind.

22. Für die Beschichtung verwendbare Zusammensetzung gemäß den Ansprüchen 11 bis 21, **dadurch gekennzeichnet, dass** sie außerdem zur nachfolgenden oder gleichzeitigen Zugabe ein Coreagenz mit reaktivem Wasserstoff umfasst.

23. Zusammensetzung gemäß den Ansprüchen 11 bis 22, **dadurch gekennzeichnet, dass** sie außerdem wenigstens einen an sich bekannten Demaskierungskatalysator für die durch Oxime maskierten lsocyanate umfasst.

24. Verwendung der Zusammensetzungen gemäß den Ansprüchen 11 bis 23 für die Vemetung einer Beschichtung.

25. Verwendung der Zusammensetzungen gemäß Anspruch 24 für die Vernetzung einer Beschichtung gemäß dem «coil coating» genannten Verfahren.

26. Verwendung der Zusammensetzungen gemäß Anspruch 24 für die Vernetzung einer Beschichtung bei einer Temperatur zwischen 110°C und 130°C.

27. Verfahren zur Herstellung von Polymeren, **dadurch gekennzeichnet, dass** es die folgenden Schritte umfasst:
□ ein geschütztes Polyisocyanat gemäß einem der Ansprüche 1 bis 10 in Gegenwart eines Coreagenzes bringen, das Derivate enthält, die reaktive Wasserstoffe aufweisen;
und
□ das so gebildete Reaktionsmedium gemäß der coil coating-Technik oder auf eine Temperatur zwischen 110°C und 130°C erhitzen.
